(19) Europäisches Patentamt
European Patent Office
Office européen des brevets

(11) Veröffentlichungsnummer: **0 123 861**
**B1**

(12) **EUROPÄISCHE PATENTSCHRIFT**

(45) Veröffentlichungstag der Patentschrift: **20.04.88**

(51) Int. Cl.⁴: **C 07 D 239/34,** A 01 N 47/34

(21) Anmeldenummer: **84102989.5**

(22) Anmeldetag: **19.03.84**

(54) **Harnstoffderivate.**

(30) Priorität: **30.03.83 DE 3311703**

(43) Veröffentlichungstag der Anmeldung: **07.11.84 Patentblatt 84/45**

(45) Bekanntmachung des Hinweises auf die Patenterteilung: **20.04.88 Patentblatt 88/16**

(84) Benannte Vertragsstaaten: **AT BE CH DE FR GB IT LI NL**

(56) Entgegenhaltungen: **DE - A - 2 123 236**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Sirrenberg, Wilhelm, Dr., Wuppertaler Strasse 21, D-4322 Sprockhövel (DE)**
Erfinder: **Maurer, Fritz, Dr., Roeberstrasse 8, D-5600 Wuppertal 1 (DE)**
Erfinder: **Klauke, Erich, Dr., Eichendorffweg 8, D-5068 Odenthal (DE)**
Erfinder: **Becker, Benedikt, Dr., Metzkausener Strasse 14, D-4020 Mettmann (DE)**
Erfinder: **Stendel, Wilhelm, Dr., In den Birken 55, D-5600 Wuppertal 1 (DE)**

EP 0 123 861 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 3-(C-Pyrimidin-5-yl-oxyphenyl)-1-benzoyl-(thio)harnstoffe, Verfahren zu ihrer Herstellung und deren Verwendung als Schädlingsbekämpfungsmittel, insbesondere als Arthropodizide, wie Insektizide.

Es ist bereits bekannt, dass bestimmte Benzoylharnstoffe, wie z.B. 1-(2,6-Difluorbenzoyl)-3-(4-chlorphenyl)-harnstoff, insektizide Eigenschaften besitzen (vgl. z.B. DE-AS 2 123 236).

Es wurden die neuen 3-(Pyrimidin-5-yl-oxyphenyl)-1-benzoyl-(thio)harnstoffe der Formel (I) gefunden,

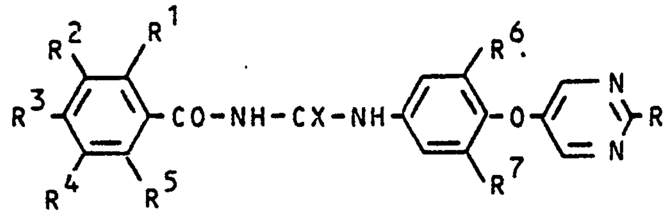

(I)

in welcher
R für Wasserstoff, $C_1$–$C_6$-Alkyl, $C_3$–$C_6$-Cycloalkyl, Halogen-$C_1$–$C_6$-Alkyl oder für einen gegebenenfalls durch Halogen, $C_1$–$C_4$-Alkyl, $C_1$–$C_4$Alkoxy, $C_1$–$C_4$-Alkylthio, Halogen-$C_1$–$C_4$-Alkyl, Halogen-$C_1$–$C_4$-Alkoxy und/oder Halogen-$C_1$–$C_4$-Alkylthio substituierten Phenylrest steht,
$R^1$ für Wasserstoff, Nitro, Halogen, $C_1$–$C_4$-Alkyl oder $C_1$–$C_4$-Alkylthio steht,
$R^2$, $R^3$, $R^4$ und $R^5$ gleich oder verschieden sind und für Wasserstoff oder Halogen stehen,
$R^6$ und $R^7$ gleich oder verschieden sind und für Wasserstoff, Halogen oder $C_1$–$C_4$-Alkyl stehen und
X für Sauerstoff oder Schwefel steht.

Diese neuen Verbindungen weisen starke biologische, insbesondere insektizide Eigenschaften auf, die ihre Verwendung als Schädlingsbekämpfungsmittel, insbesondere als Insektizide ermöglichen.

Weiterhin wurde gefunden, dass die neuen 3-(Pyrimidin-5-yl-oxy-phenyl)-1-benzoyl-(thio)harnstoffe der Formel (I) erhalten werden, indem man

a) neue substituierte Aniline der Formel (II),

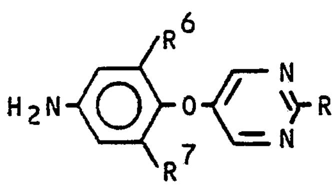

(II)

in welcher
R, $R^6$ und $R^7$ die oben angegebenen Bedeutungen haben, mit Benzoyl-iso(thio)cyanaten der Formel (III),

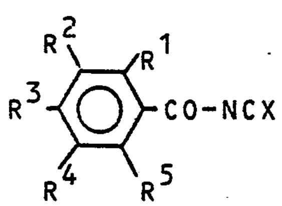

(III)

in welcher
X, $R^1$, $R^2$, $R^3$, $R^4$ und $R^5$ die oben angegebenen Bedeutungen haben, gegebenenfalls in Gegenwart eines Verdünnungsmittels umsetzt oder

b) Pyrimidinyloxy-phenyl-iso(thio)cyanate der Formel (IV)

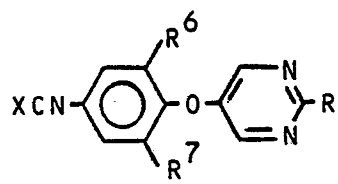

(IV)

in welcher
X, R, $R^6$ und $R^7$ die oben angegebene Bedeutungen haben, mit Benzoesäureamiden der Formel (V)

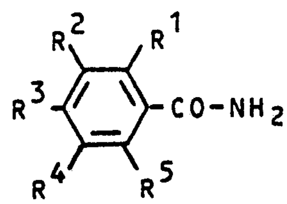

(V)

in welcher
$R^1$, $R^2$, $R^3$, $R^4$ und $R^5$ die oben angegebenen Bedeutungen haben, gegebenenfalls in Gegenwart eines Katalysators und gegebenenfalls in Gegenwart eines Verdünnungsmittels umsetzt.

Alkyl steht für geradkettiges oder verzweigtes Alkyl mit im Falle von R 1 bis 6, insbesondere 1 bis 4 Kohlenstoffatomen und im Falle von $R^1$, $R^6$ und $R^7$ mit 1 bis 4 Kohlenstoffatomen. Beispielhaft seien Methyl, Ethyl, n-Propyl, iso-Propyl, n-Butyl, iso-Butyl, sec.-Butyl und tert.-Butyl genannt.

Alkylthio $R^1$ steht für geradkettiges oder verzweigtes Alkylthio mit 1 bis 4 Kohlenstoffatomen. Beispielhaft seien Methylthio, Ethylthio, n-Propylthio, iso-Propylthio, n-Butylthio, iso-Butylthio, sec.-Butylthio und tert.-Butylthio genannt.

Cycloalkyl R steht für Cycloalkyl mit 3 bis 6 Kohlenstoffatomen, wobei beispielhaft Cyclopropyl, Cyclobutyl, Cyclopentyl und Cyclohexyl genannt seien.

Halogenalkyl R steht für Halogenalkyl mit 1 bis 6, insbesondere 1 bis 3 Kohlenstoffatomen und vorzugsweise 1 bis 5, insbesondere 1 bis 3 Halogenatomen, wobei die Halogenatome vorzugsweise Fluor, Chlor, Brom und/oder Iod, insbeson-

dere Fluor, Chlor und/oder Brom stehen, wie Trifluormethyl, Trichlormethyl, Clordifluormethyl, Dichlorfluormethyl, Trifluorethyl, Trichlorethyl, Chlordifluorethyl, Dichlorfluorethyl und n-Pentafluorpropyl.

Halogen bedeutet (wo nicht anders erläutert) Fluor, Chlor, Brom und Iod, vorzugsweise Fluor, Chlor oder Brom.

Der Phenylrest R kann einfach oder mehrfach, gleich oder verschieden durch die folgenden Substituenten substituiert sein: Halogen, wobei die Halogenatome gleich oder verschieden sind und als Halogenatome vorzugsweise Fluor, Chlor oder Brom, insbesondere Fluor oder Chlor stehen; Alkyl, Alkoxy, Alkylthio mit 1 bis 4 Kohlenstoffatomen, Halogenalkyl, Halogenalkoxy und Halogenalkylthio mit 1 bis 4, insbesondere 1 bis 3 Kohlenstoffatomen im Alkylteil und vorzugsweise 1 bis 5, insbesondere 1 bis 3 Halogenatomen, wobei die Halogenatome gleich oder verschieden sind und als Halogenatome vorzugsweise Fluor, Chlor, Brom oder Iod, insbesondere Fluor, Chlor oder Brom stehen, wie Trifluormethyl, Trifluormethoxy oder Trifluormethylthio.

Die neuen Verbindungen der Formel (I) verfügen über Eigenschaften, die ihre Verwendung als Schädlingsbekämfungsmittel ermöglichen, insbesondere zeichnen sie sich durch eine hervorragende insektizide Wirksamkeit aus.

Die Erfindung betrifft vorzugsweise neue Verbindungen der Formel (I), in welcher
R für Wasserstoff, $C_1$–$C_4$-Alkyl, $C_3$–$C_6$-Cycloalkyl, Halogen-$C_1$–$C_4$-Alkyl oder für gegebenenfalls einfach oder mehrfach durch $C_1$–$C_2$-Alkyl, $C_1$–$C_2$-Alkoxy, $C_1$–$C_2$-Alkylthio, Halogen-$C_1$–$C_2$-Alkyl, Halogen-$C_1$–$C_2$-Alkoxy, Halogen-$C_1$–$C_2$-Alkylthio, Fluor, Chlor und/oder Brom substituiertes Phenyl steht,
$R^1$ für Wasserstoff, Fluor, Chlor, Brom, Iod, Nitro, $C_1$–$C_4$-Alkyl oder $C_1$–$C_4$-Alkylthio steht,
$R^2$, $R^3$, $R^4$ und $R^5$ gleich oder verschieden sind und für Wasserstoff, Fluor, Chlor oder Brom stehen,
$R^6$ und $R^7$ gleich oder verschieden sind und für Wasserstoff, Fluor, Chlor, Brom oder $C_1$–$C_4$-Alkyl stehen und
X für Sauerstoff oder Schwefel steht.

Ganz besonders bevorzugt sind die Verbindungen der Formel (I), in welcher
R für Wasserstoff, Methyl, Ethyl, n-Propyl, i-Propyl, n-Butyl, sec.-Butyl, i-Butyl, tert.-Butyl, Cyclopropyl, Cyclohexyl oder für gegebenenfalls einfach bis dreifach durch Methyl, Ethyl, Trifluormethyl, Trifluormethoxy, Trifluormethylthio, Fluor, Chlor und/oder Brom substituiertes Phenyl steht,
$R^1$ für Fluor, Chlor, Brom oder Iod steht,
$R^2$, $R^3$, $R^4$ und $R^5$ gleich oder verschieden sind und für Wasserstoff, Fluor, Chlor oder Brom stehen,
$R^6$ und $R^7$ gleich oder verschieden sind und für Wasserstoff, Chlor oder Methyl stehen und
X für Sauerstoff oder Schwefel steht.

Verwendet man nach Verfahrensvariante (a) 3,5-Dichlor-4-(2-methyl-pyrimidin-5-yl-oxy)-anilin und 2,6-Difluor-benzoyl-isocyanat als Ausgangsstoffe, so kann der Reaktionsablauf durch das folgende Formelschema wiedergegeben werden:

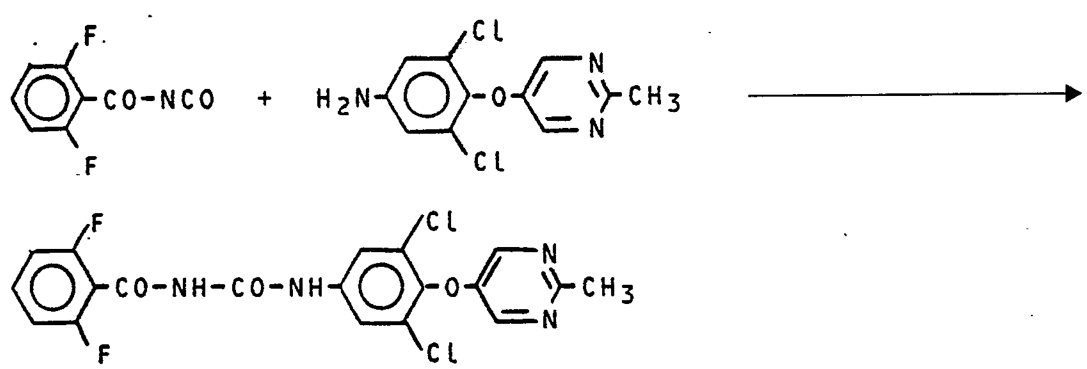

Verwendet man nach Verfahrensvariante (b) 3,5-Dichlor-4-(2-cyclopropyl-pyrimidin-5-yl-oxy)-phenyl-isocyanat und 2-Brom-benzamid als Ausgangsstoffe, so kann der Reaktionsablauf durch das folgende Formelschema wiedergegeben werden:

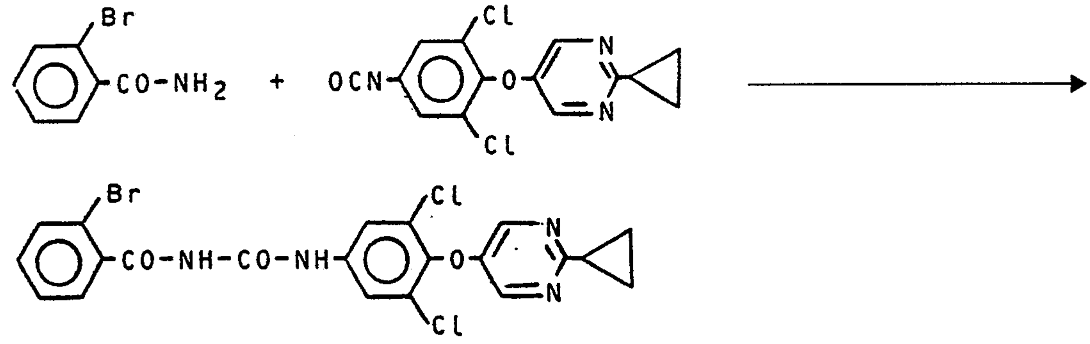

Als Beispiele für die Verbindung der Formel (II) seien genannt:

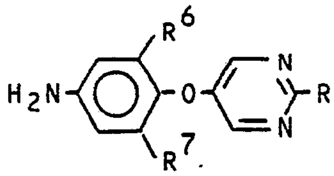

(II)

Tabelle 1

| $R^6$ | $R^7$ | R | $R^6$ | $R^7$ | R |
|---|---|---|---|---|---|
| H | H | H | H | H | 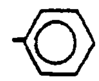 |
| H | Cl | H | H | Cl | 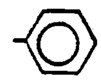 |
| H | $CH_3$ | H | H | $CH_3$ | 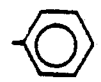 |
| Cl | Cl | H | Cl | Cl | 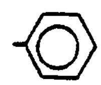 |
| Cl | $CH_3$ | H | Cl | $CH_3$ | 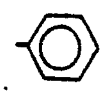 |
| $CH_3$ | $CH_3$ | H | $CH_3$ | $CH_3$ | 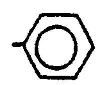 |
| H | H | $CH_3$ | H | H | 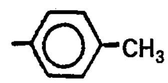 |
| H | H | tert. $C_4H_9$ | | | |
| H | Cl | tert. $C_4H_9$ | | | |
| H | Cl | $CH_3$ | Cl | Cl | 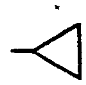 |
| H | $CH_3$ | $CH_3$ | | | |
| Cl | Cl | $CH_3$ | Cl | $CH_3$ | 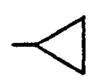 |
| Cl | $CH_3$ | $CH_3$ | | | |
| $CH_3$ | $CH_3$ | $CH_3$ | $CH_3$ | $CH_3$ | 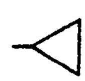 |
| H | H | $C_2H_5$ | | | |
| H | Cl | $C_2H_5$ | H | Cl | 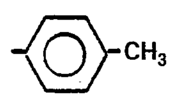 |
| H | $CH_3$ | $C_2H_5$ | | | |
| Cl | Cl | $C_2H_5$ | H | $CH_3$ | 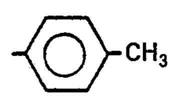 |
| Cl | $CH_3$ | $C_2H_5$ | | | |
| $CH_3$ | $CH_3$ | $C_2H_5$ | Cl | Cl | 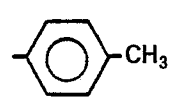 |
| H | H | n-$C_3H_7$ | | | |
| H | Cl | n-$C_3H_7$ | Cl | $CH_3$ | 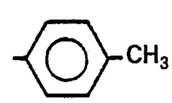 |
| H | $CH_3$ | n-$C_3H_7$ | | | |

Tabelle 1 (Fortsetzung)

| $R^6$ | $R^7$ | R | $R^6$ | $R^7$ | R |
|---|---|---|---|---|---|
| Cl | Cl | $n\text{-}C_3H_7$ | H | H | 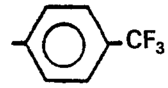 |
| Cl | $CH_3$ | $n\text{-}C_3H_7$ | | | |
| $CH_3$ | $CH_3$ | $n\text{-}C_3H_7$ | H | Cl | 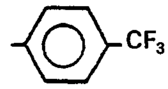 |
| H | H | $i\text{-}C_3H_7$ | | | |
| H | Cl | $i\text{-}C_3H_7$ | H | $CH_3$ | 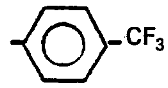 |
| H | $CH_3$ | $i\text{-}C_3H_7$ | | | |
| Cl | Cl | $i\text{-}C_3H_7$ | Cl | Cl | 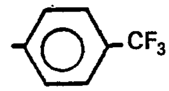 |
| Cl | $CH_3$ | $i\text{-}C_3H_7$ | | | |
| $CH_3$ | $CH_3$ | $i\text{-}C_3H_7$ | Cl′ | $CH_3$ | 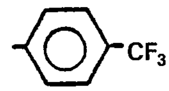 |
| H | H | 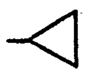 | $CH_3$ | $CH_3$ | 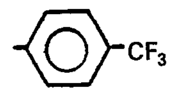 |
| H | Cl | 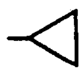 | H | H | 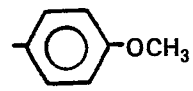 |
| H | $CH_3$ | 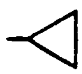 | H | Cl | 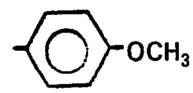 |
| H | $CH_3$ | tert. $C_4H_9$ | | | |
| H | $CH_3$ | 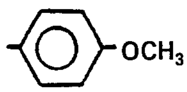 | $CH_3$ | $CH_3$ | 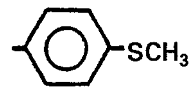 |
| Cl | Cl | 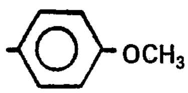 | H | H | 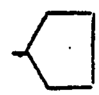 |
| Cl | $CH_3$ | 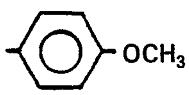 | H | Cl | 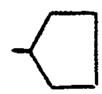 |
| $CH_3$ | $CH_3$ | 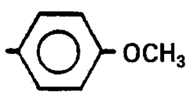 | H | $CH_3$ | 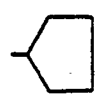 |
| H | H | 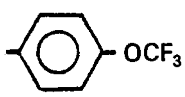 | Cl | Cl | 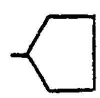 |
| H | Cl | 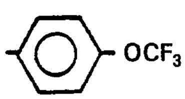 | Cl | $CH_3$ | 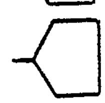 |
| H | $CH_3$ | 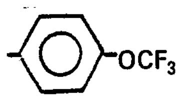 | $CH_3$ | $CH_3$ | 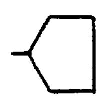 |
| Cl | Cl | 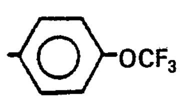 | H | H | 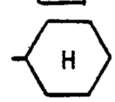 |
| Cl | $CH_3$ | 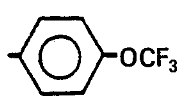 | H | Cl | 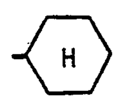 |

Tabelle 1 (Fortsetzung)

| $R^6$ | $R^7$ | R | $R^6$ | $R^7$ | R |
|---|---|---|---|---|---|
| $CH_3$ | $CH_3$ | 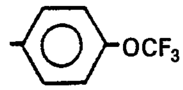 | H | $CH_3$ | 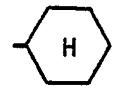 |
| H | H | 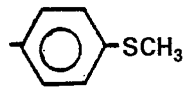 | Cl | Cl | 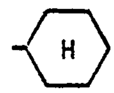 |
| H | Cl | 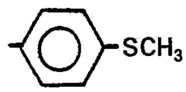 | Cl | $CH_3$ | 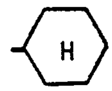 |
| H | $CH_3$ | 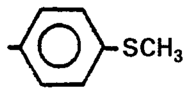 | $CH_3$ | $CH_3$ | 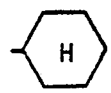 |
| Cl | Cl | 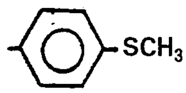 | H | H | 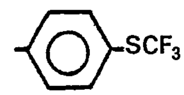 |
| Cl | $CH_3$ | 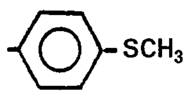 | H | Cl | 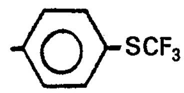 |
| Cl | Cl | tert. $C_4H_9$ | | | |
| Cl | $CH_3$ | tert. $C_4H_9$ | | | |
| H | $CH_3$ | 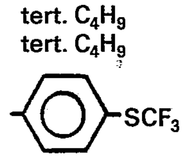 | H | Cl | 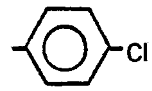 |
| Cl | Cl | 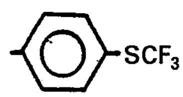 | H | $CH_3$ | 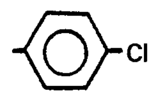 |
| Cl | $CH_3$ | 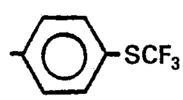 | Cl | Cl | 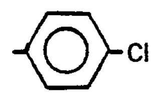 |
| $CH_3$ | $CH_3$ | 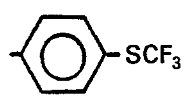 | Cl | $CH_3$ | 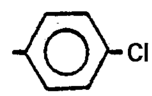 |
| H | H | 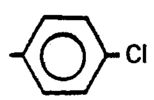 | $CH_3$ | $CH_3$ | 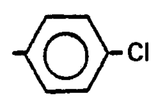 |
| $CH_3$ | $CH_3$ | tert. $C_4H_9$ | | | |

Die als Ausgangsstoffe zu verwendenden Aniline der Formel (II) sind neu. Die Herstellung der neuen Verbindungen der Formel (II) erfolgt nach bekannten Verfahren dadurch, dass man 5-Hydroxypyrimidine der Formel (VI),

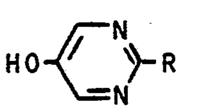 (VI)

in welcher
R die oben angegebene Bedeutung hat,
gegebenenfalls in Form der Alkali-, Erdalkali- oder Ammoniumsalze, mit Verbindungen der Formel (VII),

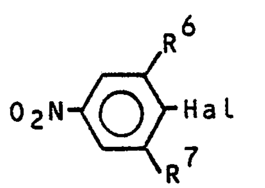 (VII)

in welcher
$R^6$ und $R^7$ die oben angegebenen Bedeutungen haben und
Hal für Halogen, insbesondere Chlor oder Brom steht,

gegebenenfalls in Gegenwart von Verdünnungsmitteln, wie z.B. Dimethylsulfoxid, gegebenenfalls in Gegenwart von Säureakzeptoren, wie z.B. Kaliumcarbonat, Kaliumhydroxid oder Natriumhydroxid bei Temperaturen zwischen 50°C

und 200°C umsetzt und die auf diese Weise hergestellten neuen Verbindungen der Formel (VIII),

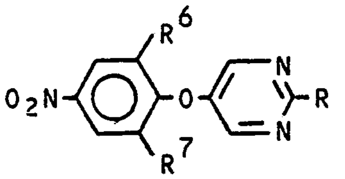

(VIII)

in welcher

R, $R^6$ und $R^7$ die oben angegebenen Bedeutungen haben, nach üblichen Methoden, z.B. durch Reduktion im sauren Medium mit Reaktionsmitteln wie Zinn-II-chlorid und Salzsäure, gegebenenfalls in Gegenwart von Lösungsmitteln wie Ethanol, bei Temperaturen zwischen −10°C und +100°C zu den entsprechenden neuen Anilinen der Formel (II) reduziert (vgl. Herstellungsbeispiele).

Die Verbindungen der Formel (VI) sind bekannt und nach literarbekannten Verfahren und Methoden herstellbar (vgl. z.B. DE 2 634 262, DE 2 706 127 und DE 2 835 492).

Die Verbindungen der Formel (VII) sind allgemein bekannte Verbindungen der organischen Chemie.

Die Verbindungen der Formel (VIII) sind neu und lassen sich nach den oben angegebenen Verfahren herstellen. Als Beispiele für die Verbindungen der Formel (VIII) seien die entsprechenden Nitro-Derivate der Verbindungen der Formel (II) in Tabelle 1 genannt.

Als Beispiele für die Verbindungen der Formel (III) seien genannt:

2-Fluor-, 2-Chlor-, 2-Brom-, 2-lod-, 2-Nitro-, 2-Methyl-, 2-Methyltio-, 2,4-Dichlor-, 2,4-Difluor, 2,4-Dibrom-, 2,4-Dimethyl-, 2-Chlor-4-fluor-, 4-Chlor-2-fluor-, 3-Chlor-4-fluor-, 3,4-Dichlor-, 4-Chlor-, 4-Fluor-, 4-Brom-, 2-Chlor-5-fluor-, 2-Brom-4-fluor-, 2,5-Dichlor-, 2,5-Difluor-, 5-Chlor-2-fluor, 2,6-Difluor-, 2,6-Dichlor-, 2,6-Dibrom-, 2-Chlor-6-fluor-, 2-Brom-6-fluor-, 2-Methyl-6-fluor-, 2-Methylthio-6-fluor-benzoyl-isocyanat- bzw. -benzoyl-iso-thiocyanat.

Die Benzoyl-iso(thio)cyanate der Formel (III) sind bekannt.

Die für die Verfahrensvariante (b) zu verwendeten Pyrimidinyloxy-phenyl-iso(thio)cyanate sind durch die Formel (IV) definiert.

Die Pyrimidinyloxy-phenyl-iso(thio)cyanate der Formel (IV) sind neu. Sie lassen sich nach allgemeinen üblichen Methoden aus den entsprechenden neuen substituierten Anilinen der Formel (II) herstellen (vgl. z.B. EP 0 057 888).

Als Beispiele für die Ausgangsverbindungen der Formel (V) seien genannt:

2-Fluor, 2-Chlor-, 2-Brom-, 2-lod-, 2-Nitro-, 2-Methyl-, 2-Methyltio-, 2,4-Dichlor-, 2,4-Difluor-, 2,4-Dibrom-, 2,4-Dimethyl-, 2-Chlor-4-fluor-, 4-Chlor-2-fluor, 3-Chlor-4-fluor-, 3,4-Dichlor-, 4-Chlor-, 4-Brom-, 4-Fluor-, 2-Chlor-5-fluor-, 2-Brom-4-fluor-, 2,4-Dichlor-, 2,5-Difluor-, 5-Chlor-2-fluor-, 2,6-Difluor-, 2,6-Dichlor-, 2,6-Dibrom-, 2-Chlor-6-fluor-, 2-Brom-6-fluor-, 2-Methyl-6-fluor- und 2-Methylthio-6-fluor-benzoesäureamid.

Die Verbindungen der Formel (V) sind bekannt oder können nach allgemein bekannten Methoden und Verfahren erhalten werden (vgl. z.B. Z. Obsc. Chim. 11 (1941) 243; C.A. 1941, 7965).

Als Verdünnungsmittel für die Verfahrensvarianten (a) und (b) kommen praktisch alle inerten organischen Lösungsmittel infrage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Yxlol, Methylenchlorid, Ethylenchlorid, Chloroform, Tetrachlorkohlenstoff, Chlorbenzol und o-Dichlorbenzol, Ether wie Diehtyl- und Dibutylether, Glykoldimethylether und Diglykoldimethylether, Tetrahydrofuran und Dioxan, Ketone, wie Aceton, Methyl-ethyl-, Methyl-isopropyl- und Methylisobutylketon, Ester, wie Essigsäuremethylester und -ethylester, Nitrile, wie z.B. Acetonitril und Propionitril, Amide, wie z.B. Dimethylacetamid und N-Methyl-pyrrolidon, sowie Tetramethylensulfon.

Als Katalysatoren können für die Umsetzung gemäss Verfahrensvariante (b) vorzugsweise tertiäre Amine, wie Triethylamin und 1,4-Diazabicyclo [2,2,2]-octan, sowie organische Zinn-Verbindungen, wie z.B. Dibutylzinndilaurat verwendet werden.

Die Reaktionstemperatur kann innerhalb eines grösseren Bereiches variiert werden. Im allgemeinen arbeitet man bei der Verfahrensvariante a) zwischen 20 und 180°C, vorzugsweise zwischen 60 und 120°C und bei der Verfahrensvariante b) zwischen 20 und 200°C, vorzugsweise zwischen 60 und 190°C. Die erfindungsgemässen Verfahrensvarianten werden im allgemeinen bei Normaldruck durchgeführt.

Zur Durchführung der erfindungsgemässen Verfahrensvarianten (a) und (b) werden die Ausgangsstoffe gewöhnlich in etwa äquimolaren Mengen eingesetzt. Ein Überschuss der einen oder anderen Reaktionskomponente bringt keine wesentlichen Vorteile.

Die Aufarbeitung der Reaktionsprodukte geschieht nach üblichen Methoden, z.B. durch Absaugen des ausgefallenen Produktes oder durch Herauslösen von unerwünschten Nebenprodukten aus dem Reaktionsgemisch. Zur Charakterisierung dient der Schmelzpunkt.

Die Herstellung der Verbindungen der Formel (I) wird bevorzugt nach Verfahrensvariante (a) durchgeführt.

Die vorliegende Erfindung betrifft ausser den Verbindungen der Formel (I), ihre Herstellung und Verwendung auch die Zwischenprodukte der Formel (II), (IV) und (VIII), welche in der Formel (IX) zusammengefasst werden können:

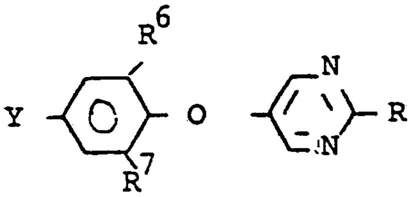

(IX)

in welcher
R für Wasserstoff, $C_1$–$C_6$-Alkyl, $C_3$–$C_6$-Cycloalkyl, Halogen-$C_1$–$C_6$-Alkyl oder für einen gegebenenfalls durch Halogen, $C_1$–$C_4$-Alkyl, $C_1$–$C_4$-Alkoxy, $C_1$–$C_4$Alkylthio, Halogen-$C_1$–$C_4$-Alkyl, Halogen-$C_1$–$C_4$-Alkoxy und/oder Halogen-$C_1$–$C_4$-Alkylthio substituierten Phenylrest steht,
$R^6$ und $R^7$ gleich oder verschieden sind für Wasserstoff, Halogen oder $C_1$–$C_4$-Alkyl stehen und
Y für $NH_2$, $NO_2$ oder NCX (vorzugsweise für $NH_2$) steht, wobei X Sauerstoff oder Schwefel bedeutet.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina. Aus der Ordnung der Collembola z.B. Onychiurus armatus. Aus der Ordnung der Orthoptera z.B. Blatta orientalis, Periplaneta americana, Leucophaea maderae, Blattella germanica, Acheta domesticus, Gryllotalpa spp., Locusta migratoria migratoriodes, Melanoplus differentialis, Schistocerca gregaria.

Aus der Ordnung der Dermaptera z.B. Forficula auricularia.

Aus der Ordnung der Isoptera z.B. Reticultitermes spp.. Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinotthrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicornyne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thruberiella, Phyllocnistis citrella, Agrotis spp., Euxoa spp., Feltia spp., Earias insulana, Heliothis spp., Laphygma exigua, Mamestra brasssicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana. Aus der Ordnung der Cleoptera z.B. Anobium punctatum, Rhizopertha dominica, Bruchidius obtectus, Acanthoscelides obtenctus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hypoposca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans. Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter

Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Menatiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemässen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.% Wirkstoff, vorzugsweise zwischen 0,0001 und 1 Gew.% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemässen Wirkstoffe eignen sich auch zur Bekämpfung von Ekto- und Endoparasiten auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemässen Wirkstoffe geschieht auf diesen Gebieten in bekannter Weise, wie durch äusserliche Anwendung in Form beispielsweise des Tauchens (Dippen), Sprühens (Sprayen), Aufgiessens (pour-on and spot-on) und des Eindpuderns sowie durch orale Anwendung, beispielsweise über das Futter oder Trinkwasser in Form von beispielsweise Tabletten, Kapseln, Tränken, Granulaten.

Die Herstellung der neuen Verbindungen der Formel (I) soll anhand der folgenden Herstellungsbeispiele erläutert werden:

Beispiel 1

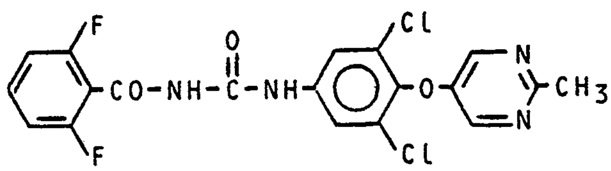

(Verfahrensvariante a)

Zu einer Lösung von 4,05 g (0,015 Mol) 3,5-Dichlor-4-(2-methyl-pyrimidin-5-yl-oxy)-anilin in 60 ml trockenem Toluol tropft man bei 60°C 2,75 g (0,015 Mol) 2,6-Difluorbenzoylisocyanat in 10 ml Toluol. Anschliessend wird eine Stunde bei 80°C gerührt. Nach Abkühlen auf 20°C wird abgesaugt und getrocknet.

Man erhält 6,0 g (99,5% der Theorie) N-[3,5-Dichlor-4-(2-methyl-pyrimidin-5-yl-oxy)-phenyl]-N'-(2,6-difluor-benzoyl)-harnstoff mit einem Schmelzpunkt Fp.: 263°C.

Beispiel 2

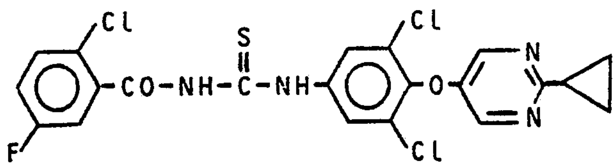

(Verfahrensvariante b)

Zu einer Lösung von 2,96 g (0,01 Mol) 3,5-Dichlor-4-(2-cyclopropyl-pyrimidin-5-yl-oxy)-anilin in 50 ml trockenem Toluol wird bei 60°C eine Lösung von 21,6 g (0,01 Mol) 2-chlor-5-fluor-benzoyl-isothiocyanat in 10 ml Toluol zugetropft. Der Ansatz wird 30 Minuten bei 80°C gerührt und anschliessend auf 20°C abgekühlt. Durch Zugabe von Petrolether wird das Reaktionsprodukt ausgefällt. Anschliessend wird abgesaugt und getrocknet.

Man erhält 5,0 g (97,5% der Theorie) N-[3,5-Dichlor-4-(2-cyclopropyl-pyrimidin-5-yl-oxy)-phenyl]-N'-(3,5-dichlor-benzoyl)-harnstoff vom Schmelzpunkt Fp.: 176°C.

Analog Beispiel 1 und 2 können die in der nachstehenden Tabelle 2 aufgeführten Verbindungen der Formel (I) hergestellt werden:

Tabelle 2

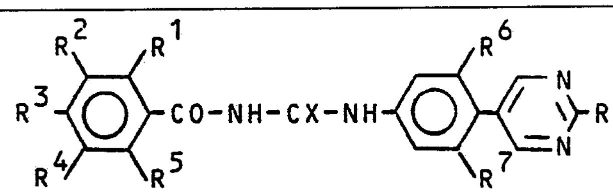

(I)

| Beisp. Nr. | $R^1$ | $R^2$ | $R^3$ | $R^4$ | $R^5$ | $R^6$ | $R^7$ | R | X | Fp./°C |
|---|---|---|---|---|---|---|---|---|---|---|
| 3 | H | Cl | F | H | H | Cl | Cl | $CH_3$ | O | 224 |
| 4 | F | H | Cl | H | H | Cl | Cl | $CH_3$ | O | 258 |
| 5 | F | H | H | H | F | H | H | $CH_3$ | O | 215 |
| 6 | Cl | H | H | H | F | H | H | $CH_3$ | O | 200 |
| 7 | Cl | H | H | H | F | Cl | Cl | $CH_3$ | O | 218 |
| 8 | Cl | H | H | H | H | Cl | Cl | 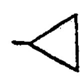 | O | 193 |
| 9 | F | H | H | H | F | Cl | Cl | 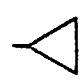 | O | 189 |
| 10 | Cl | H | H | H | F | Cl | Cl | 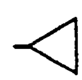 | O | 222 |
| 11 | I | H | H | H | H | Cl | Cl | 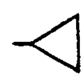 | O | 202 |
| 12 | F | H | H | H | F | H | H | 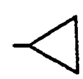 | O | 202 |
| 13 | Cl | H | H | H | Cl | H | H | 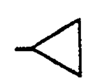 | O | 199 |

Tabelle 2 (Fortsetzung)

| Beisp. Nr. | $R^1$ | $R^2$ | $R^3$ | $R^4$ | $R^5$ | $R^6$ | $R^7$ | R | X | Fp./°C |
|---|---|---|---|---|---|---|---|---|---|---|
| 14 | Br | H | H | H | H | H | H | 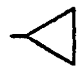 | O | 173 |
| 15 | Cl | H | F | H | H | Cl | Cl | 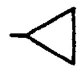 | O | 220 |
| 16 | Cl | H | H | F | H | Cl | Cl | 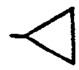 | O | 197 |
| 17 | Br | H | H | H | H | Cl | Cl | 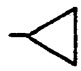 | O | 195 |
| 18 | $NO_2$ | H | H | H | H | Cl | Cl | 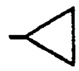 | O | 218 |
| 19 | $CH_3$ | H | H | H | H | Cl | Cl | 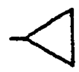 | O | 210 |
| 20 | F | H | H | H | F | Cl | Cl | 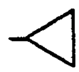 | S | 224 |
| 21 | Br | H | H | H | H | Cl | Cl | 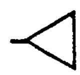 | S | 191 |
| 22 | H | Cl | Cl | H | H | Cl | Cl | 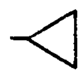 | O | 234 |
| 23 | $SCH_3$ | H | H | H | H | Cl | Cl | 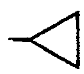 | O | 184 |
| 24 | H | H | Cl | H | H | Cl | Cl | 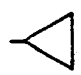 | O | 268 |
| 25 | Cl | H | F | H | H | Cl | Cl | 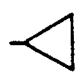 | S | 190 |
| 26 | F | H | H | H | F | Cl | Cl | 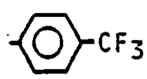 | O | 193 |
| 27 | Cl | H | H | H | H | Cl | Cl | 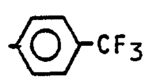 | O | 202 (Zers.) |
| 28 | Cl | H | H | H | F | Cl | Cl | 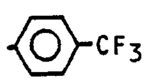 | S | 217 (Zers.) |
| 29 | F | H | H | H | F | $CH_3$ | $CH_3$ | 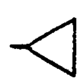 | S | 206–207 |
| 30 | Cl | H | F | H | H | $CH_3$ | $CH_3$ | 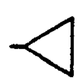 | S | 177 |

Tabelle 2 (Fortsetzung)

| Beisp. Nr. | R¹ | R² | R³ | R⁴ | R⁵ | R⁶ | R⁷ | R | X | Fp./°C |
|---|---|---|---|---|---|---|---|---|---|---|
| 31 | Br | H | H | H | H | $CH_3$ | $CH_3$ | 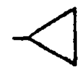 | S | 201 |
| 32 | $CH_3$ | H | H | H | H | $CH_3$ | $CH_3$ | 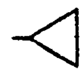 | O | 202 |
| 33 | Cl | H | H | H | H | $CH_3$ | $CH_3$ | 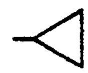 | O | 177 |
| 34 | F | H | H | H | F | $CH_3$ | $CH_3$ | 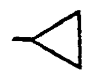 | O | 188 |
| 35 | Cl | H | H | H | F | $CH_3$ | $CH_3$ | 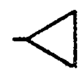 | O | 219 |
| 36 | Cl | H | H | H | Cl | $CH_3$ | $CH_3$ | 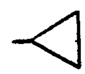 | O | 221–222 |
| 37 | Cl | H | H | H | H | Cl | Cl | 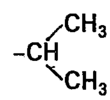 | O | 187 |
| 38 | Cl | H | F | H | H | Cl | Cl | 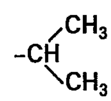 | O | 212–213 |
| 39 | F | H | H | H | F | Cl | Cl | 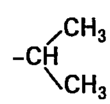 | O | 200 |
| 40 | F | H | H | H | F | Cl | Cl | 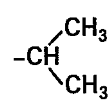 | S | 177–179 |
| 41 | F | H | H | H | F | Cl | Cl | 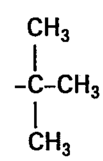 | O | 193 |
| 42 | Cl | H | F | H | H | $CH_3$ | $CH_3$ | 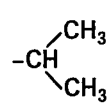 | O | 171 |

Ausgangsprodukte der Formel (II):

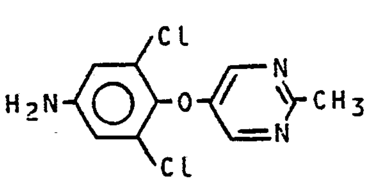

(II-1)

Zu einer Aufschlämmung von 45 g (0,15 Mol) 3,5-Dichlor-4-(2-methyl-pyrimidin-5-yl-oxy)-nitrobenzol und 225 ml Ethanol wird bei 0°C bis 5°C eine Lösung von 101 g Zinn-II-chlorid-dihydrat in 190 ml konzentrierte Salzsäure getropft. Nachdem die Temperatur auf 20°C angestiegen ist, wird 2 Stunden bei 50°C bis 60°C gerührt. Nach Abkühlung auf 20°C wird in Eiswasser eingegossen, mit konzentrierter Natronlauge (200 g Natriumhydroxid und 300 ml Wasser) versetzt und anschliessend zweimal mit Ethylenchlorid extrahiert, dreimal mit Wasser gewaschen und getrocknet. Nach Entfernen des Lösungsmittels

erhält man 38,2 g (94% der Theorie) 3,5-Dichlor-4-(2-methyl-pyrimidin-5-yl-oxy)-anilin vom Schmelzpunkt Fp.: 147°C.

Analog Beispiel (II-1) wurden die übrigen in Tabelle 3 aufgeführten Verbindungen der Formel (II) hergestellt:

Tabelle 3

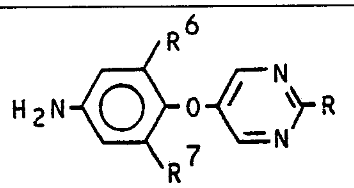

(II)

| Beisp. Nr. | $R^6$ | $R^7$ | R | Schmelzpunkt Fp./°C |
|---|---|---|---|---|
| II-2 | H | H | $CH_3$ | 115 |
| II-3 | H | H | 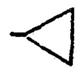 | 97 |
| II-4 | Cl | Cl | 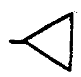 | 137 |
| II-5 | $CH_3$ | $CH_3$ | $-C_3H_7$-iso | 87 |
| II-6 | Cl | Cl | $-C_3H_7$-iso | 111 |
| II-7 | $CH_3$ | $CH_3$ | 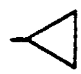 | 137 |
| II-8 | Cl | Cl | $-C_3H_7$-iso | |
| II-9 | Cl | Cl | 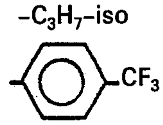 | 98 |
| II-10 | Cl | Cl | $-C_4H_9$-tert. | 143–145 |

– Die Reduktion der Nitroverbindungen der Formel (VIII) zu Verbindungen der Formel (II) kann auch mittels Wasserstoff mit Hilfe von Katalysatoren durchgeführt werden.

Ausgangsprodukte der Formel (VIII):

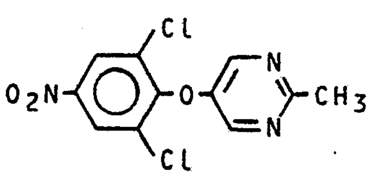

(VIII-1)

22 g (0,2 Mol) 2-Methyl-5-hydroxy-pyrimidin werden in 180 ml Dimethylsulfoxid gelöst und eine Lösung von 13 g Kaliumhydroxid in 10 ml Wasser zugefügt. Der Ansatz wird 30 Minuten bei 60°C gerührt. Zur Entfernung des entstandenen Wassers werden ca. 40 ml unter Vakuum abdestilliert. Der Rückstand wird mit einer Lösung von 45,4 g (0,2 Mol) 3,4,5-Trichlor-nitrobenzol in 80 ml Dimethylsulfoxid versetzt und 4 Stunden bei 80°C bis 100°C gerührt und anschliessend auf 20°C abgekühlt. Nach Absaugen des festen Reaktionsproduktes wird mehrfach mit Wasser gewaschen und schliesslich aus wässrigem Methanol umkristallisiert.

Man erhält 37,3 g (62% der Theorie) 3,5-Dichlor-4-(2-methyl-pyrimidin-5-yl-oxy)-nitrobenzol vom Schmelzpunkt Fp.: 109°C.

Analog Beispiel (VIII-1) werden die übrigen in Tabelle 4 aufgeführten Verbindungen der Formel (VIII) hergestellt:

Tabelle 4

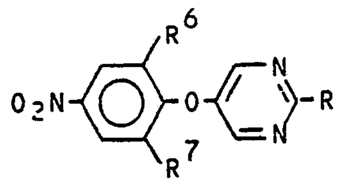

(VIII)

| Beisp. Nr. | $R^6$ | $R^7$ | R | Schmelzpunkt Fp./°C bzw. Brechungsindex $n_D^{20}$ |
|---|---|---|---|---|
| VIII-2 | H | H | $CH_3$ | 110 |
| VIII-3 | H | H | 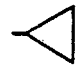 | 81 |
| VIII-4 | Cl | Cl | 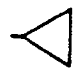 | 107 |
| VIII-5 | $CH_3$ | $CH_3$ | $-C_3H_7$-iso | 101 |
| VIII-6 | Cl | Cl | $-C_3H_7$-iso | $n_D^{20}$: 1,5915 |
| VIII-7 | $CH_3$ | $CH_3$ | 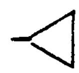 | 135 |
| VIII-8 | Cl | Cl | $-C_3H_7$-iso | |
| VIII-9 | Cl | Cl | 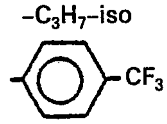 | 131 |
| VIII-10 | Cl | Cl | $-C_4H_9$-tert. | zähes Öl |

Die biologische Wirksamkeit der neuen Verbindungen soll anhand der folgenden Beispiele erläutert werden:

Beispiel A

Plutella-Test

Lösungsmittel: 15 Gewichtsteile Dimethylformamid

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirktstoffbereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, dass alle Raupen abgetötet wurden. 0% bedeutet, dass keine Raupen abgetötet wurden.

Bei diesem Test zeigen z.B. bei einer Wirkstoffkonzentration von 0,001% nach 7 Tagen beispielsweise die Verbindungen der Herstellungsbeispiele (1), (8), (9), (10), (12), (17), (20) und (21) einen Abtötungsgrad von 100%.

Beispiel B

Test mit Lucilia cuprina resistent-Larven

Emulgator: 35 Gewichtsteile Ethylenglykolmonomethylether

35 Gewichtsteile Nonylphenolpolyglykolether

Zur Herstellung einer zweckmässigen Wirkstoffzubereitung vermischte man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei einem Test z.B. mit einer Wirkstoffkonzentration von 1000 ppm zeigte beispielsweise die Verbindung des Herstellungsbeispiels (7) eine 100%ige Abtötung.

**Patentansprüche**

1. 3-(Pyrimidin-5-yl-oxy-phenyl)-1-benzoyl-(thio)harnstoffe der Formel (I)

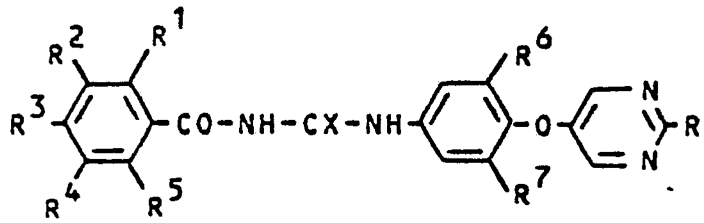

(I)

in welcher
R für Wasserstoff, $C_1$–$C_6$-Alkyl, $C_3$-$C_6$-Cycloalkyl, Halogen-$C_1$-$C_6$-Alkyl oder für einen gegebenenfalls durch Halogen, $C_1$–$C_4$-Alkyl, $C_1$–$C_4$-Alkoxy, $C_1$–$C_4$-Alkylthio, Halogen-$C_1$–$C_4$-Alkyl, Halogen-$C_1$–$C_4$-Alkoxy und/oder Halogen-$C_1$–$C_4$-Alkylthio substituierten Phenylrest steht,
$R^1$ für Wasserstoff, Nitro, Halogen, $C_1$–$C_4$-Alkyl oder $C_1$–$C_4$-Alkylthio steht,
$R^2$, $R^3$, $R^4$ und $R^5$ gleich oder verschieden sind und für Wasserstoff oder Halogen stehen,
$R^6$ und $R^7$ gleich oder verschieden sind und für Wasserstoff, Halogen oder $C_1$–$C_4$-Alkyl stehen und
X für Sauerstoff oder Schwefel steht.

2. Verbindungen der Formel (I) gemäss Anspruch 1 in welchen
R für Wasserstoff, $C_1$–$C_4$-Alkyl, $C_3$–$C_6$-Cycloalkyl, Halogen-$C_1$–$C_4$-Alkyl oder für gegebenenfalls einfach oder mehrfach durch $C_1$–$C_2$-Alkyl, $C_1$–$C_2$-Alkoxy, $C_1$–$C_2$-Alkylthio, Halogen-$C_1$–$C_2$-Alkyl, Halogen-$C_1$–$C_2$-Alkoxy, Halogen-$C_1$–$C_2$-Alkylthio, Fluor, Chlor und/oder Brom substituiertes Phenyl steht,
$R^1$ für Wasserstoff, Fluor, Chlor, Brom, Iod, Nitro, $C_1$–$C_4$-Alkyl oder $C_1$–$C_4$-Alkylthio steht,
$R^2$, $R^3$, $R^4$ und $R^5$ gleich oder verschieden sind und für Wasserstoff, Fluor, Chlor oder Brom stehen,
$R^6$ und $R^7$ gleich oder verschieden sind und für Wasserstoff, Fluor, Chlor, Brom oder $C_1$–$C_4$-Alkyl stehen und
X für Sauerstoff oder Schwefel steht.

3. Verbindungen der Formel (I) gemäss Anspruch 1, in welchen
R für Wasserstoff, Methyl, Ethyl, n-Propyl, i-Propyl, n-Butyl, sec.-Butyl, i-Butyl, ter.-Butyl, Cyclopropyl, Cyclophexyl oder für gegebenenfalls einfach bis dreifach durch Methyl, Ethyl, Trifluormethyl, Trifluormethoxy, Trifluormethylthio, Fluor, Chlor und/oder Brom substituiertes Phenyl steht,
$R^1$ für Fluor, Chlor, Brom oder Iod steht,
$R^2$, $R^3$, $R^4$ und $R^5$ gleich oder verschieden sind und für Wasserstoff, Fluor, Chlor oder Brom stehen,
$R^6$ und $R^7$ gleich oder verschieden sind und für Wasserstoff, Chlor oder Methyl stehen und
X für Sauerstoff oder Schwefel steht.

4. Verfahren zur Herstellung von 3-(Pyrimidin-5-yl-oxy-phenyl)-1-benzoyl-(thio)harnstoffen der Formel (I)

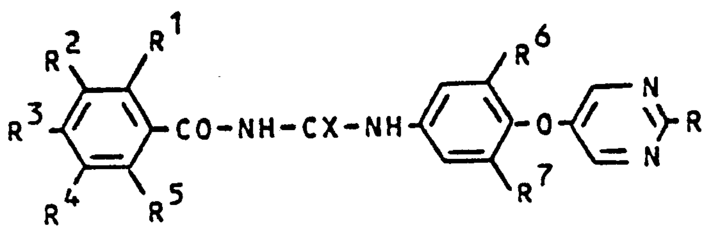

(I) (I)

in welcher
R für Wasserstoff, $C_1$–$C_6$-Alkyl, $C_3$-$C_6$-Cycloalkyl, Halogen-$C_1$-$C_6$-Alkyl oder für einen gegebenenfalls durch Halogen, $C_1$–$C_4$-Alkyl, $C_1$–$C_4$-Alkoxy, $C_1$–$C_4$-Alkylthio, Halogen-$C_1$–$C_4$-Alkyl, Halogen-$C_1$–$C_4$-Alkoxy und/oder Halogen-$C_1$–$C_4$-Alkylthio substituierten Phenylrest steht,
$R^1$ für Wasserstoff, Nitro, Halogen, $C_1$–$C_4$-Alkyl oder $C_1$–$C_4$-Alkylthio steht,
$R^2$, $R^3$, $R^4$ und $R^5$ gleich oder verschieden sind und für Wasserstoff oder Halogen stehen,
$R^6$ und $R^7$ gleich oder verschieden sind und für Wasserstoff, Halogen oder $C_1$–$C_4$-Alkyl stehen und
X für Sauerstoff oder Schwefel steht,
dadurch gekennzeichnet, dass man
a) substituierte Aniline der Formel (II),

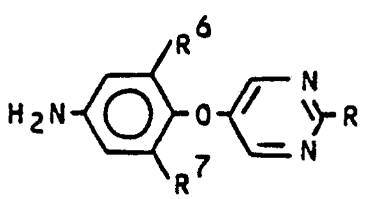

(II)

in welcher
R, $R^6$ und $R^7$ die oben angegebene Bedeutung haben, mit Benzoyl-iso(thio)cyanaten der Formel (III),

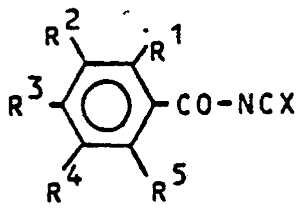

(III)

in welcher
X, $R^1$, $R^2$, $R^3$, $R^4$ und $R^5$ die oben angegebene Bedeutung haben,
gegebenenfalls in Gegenwart eines Verdünnungsmittels umsetzt oder

b) Pyrimidinyloxy-phenyl-iso(thio)cyanate der Formel (IV)

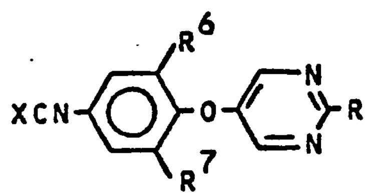

(IV)

in welcher
X, R, $R^6$ und $R^7$ die oben angegebenen Bedeutungen haben, mit Benzosäureamiden der Formel (V)

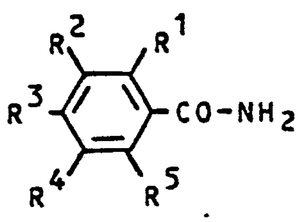

(V)

in welcher
$R^1$, $R^2$, $R^3$, $R^4$ und $R^5$ die oben angegebenen Bedeutungen haben, gegebenenfalls in Gegenwart eines Katalysators und gegebenenfalls in Gegenwart eines Verdünnungsmittels umsetzt.

5) Schädlingsbekämpfungsmittel, gekennzeichnet durch einen Gehalt an mindestens einer Verbindung der Formel (I) gemäss Anspruch 1 oder 4.

6) Verwendung von Verbindungen der Formel (I) gemäss Anspruch 1 oder 4 zur Bekämpfung von Schädlingen, insbesondere von Insekten.

7) Verfahren zur Bekämpfung von Schädlingen, dadurch gekennzeichnet, dass man Verbindungen der Formel (I) gemäss Anspruch 1 oder 4 auf die Schädlinge, vorzugsweise Insekten oder ihren Lebensraum einwirken lässt.

8) Verfahren zur Herstellung von Schädlingsbekämpfungsmitteln, dadurch gekennzeichnet, dass man Verbindungen der Formel (I) gemäss Anspruch 1 oder 4 mit Streckmitteln und/oder oberflächenaktiven Mitteln vermischt.

9) Verbindungen der Formel (IX)

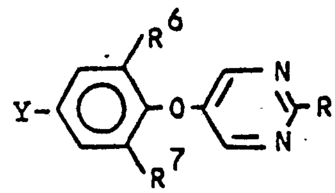

(IX)

in welcher
R für Wasserstoff, $C_1$–$C_6$-Alkyl, $C_3$–$C_6$-Cycloalkyl, Halogen-$C_1$–$C_6$-Alkyl oder für einen gegebenenfalls durch Halogen, $C_1$–$C_4$-Alkyl, $C_1$–$C_4$-Alkoxy, $C_1$–$C_4$-Alkylthio, Halogen-$C_1$–$C_4$-Alkyl, Halogen-$C_1$–$C_4$-Alkoxy und/oder Halogen-$C_1$–$C_4$-Alkylthio substituierten Phenylrest steht,
$R^6$ und $R^7$ gleich oder verschieden sind und für Wasserstoff, Halogen oder $C_1$–$C_4$-Alkyl stehen und
Y für $NO_2$, $NH_2$ oder NCX steht, wobei X Sauerstoff oder Schwefel bedeutet.

## Claims

1. 3-(Pyrimidin-5-yl-oxy-phenyl)-1-benzoyl-(thio)-ureas of the formula (I)

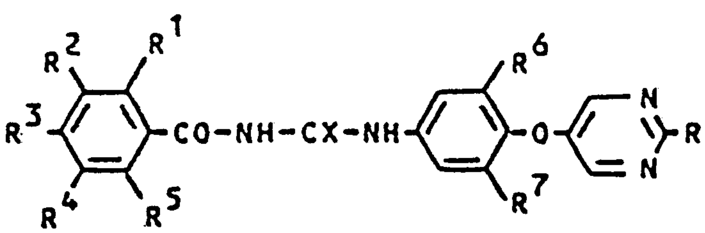

(I)

in which
R respresents hydrogen, $C_1$–$C_6$-alkyl, $C_3$–$C_6$-cycloalkyl, halogeno-$C_1$–$C_6$-alkyl or a phenyl radical which is optionally substituted by halogen, $C_1$–$C_4$-alkyl, $C_1$–$C_4$-alkoxy, $C_1$–$C_4$-alkylthio, halogeno-$C_1$–$C_4$-alkyl, halogeno-$C_1$–$C_4$-alkoxy and/or halogeno-$C_1$–$C_4$-alkylthio, $R^1$ represents hydrogen, nitro, halogen, $C_1$–$C_4$-alkyl or $C_1$–$C_4$-alkylthio,
$R^2$, $R^3$, $R^4$, and $R^5$ are identical or different and represent hydrogen or halogen,
$R^6$ and $R^7$ are identical or different and represent hydrogen, halogen or $C_1$–$C_4$-alkyl and
X represents oxygen or sulphur.

2. Compounds of the formula (I) according to Claim 1 in which
R represents hydrogen, $C_1$–$C_4$-alkyl, $C_3$–$C_6$-cycloalkyl, halogeno-$C_1$–$C_4$-alkyl, or phenyl which is optionally monosubstituted or polysubstituted by $C_1$–$C_2$-alkyl, $C_1$–$C_2$-alkoxy, $C_1$–$C_2$-alkylthio, halogene-$C_1$–$C_2$-alkyl, halogeno-$C_1$–$C_2$-alkoxy, halogeno-$C_1$–$C_2$-alkylthio, fluorine, chlorine and/or bromine,
$R^1$ represents hydrogen, fluorine, chlorine, bromine, iodine, nitro, $C_1$–$C_4$-alkyl or $C_1$–$C_4$-alkylthio,
$R^2$, $R^3$, $R^4$ and $R^5$ are identical or different and represent hydrogen, fluorine, chlorine or bromine,

$R^6$, $R^7$ are identical or different and represent hydrogen, fluorine, chlorine, bromine or $C_1$–$C_4$-alkyl and
X represents oxygen or sulphur.

3. Compounds of the formula (I) according to Claim 1 in which
R represents hydrogen, methyl, ethyl, n-propyl, i-propyl, n-butyl, sec.-butyl, i-butyl, tert.-butyl, cyclopropyl, cyclohexyl, or phenyl which is optionally monosubstituted to trisubstituted by methyl, ethyl, trifluormethyl, trifluoromethoxy, trifluormethylthio, fluorine, chlorine and/or bromine,
$R^1$ represents fluorine, chlorine, bromine or iodine,
$R^2$, $R^3$, $R^4$ and $R^5$ are identical or different and represent hydrogen, fluorine, chlorine or bromine,
$R^6$ and $R^7$ are identical or different and represent hydrogen, chlorine or methyl and X represents oxygen or sulphur.

4. Process for the preparation of 3-(pyrimidin-5-yl-oxy-phenyl)-1-benzoyl-(thio)ureas of the formula (I)

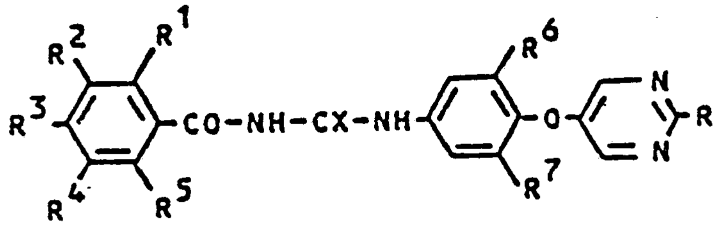 (I)

in which
R represents hydrogen, $C_1$–$C_6$-alkyl, $C_3$–$C_6$-cycloalkyl, halogeno$C_1$–$C_6$-alkyl or a phenyl radical which is optionally substituted by halogen. $C_1$–$C_4$-alkyl, $C_1$–$C_4$-alkoxy, $C_1$–$C_4$-alkylthio, halogeno-$C_1$–$C_4$-alkyl, halogeno-$C_1$–$C_4$-alkoxy and/or halogeno-$C_1$–$C_4$-alkylthio,
$R^1$ represents hydrogen, nitro, halogen, $C_1$–$C_4$-alkyl or $C_1$–$C_4$-alkylthio,
$R^2$, $R^3$, $R^4$ and $R^5$ are identical or different and represent hydrogen or halogen,
$R^6$ and $R^7$ are identical or different and represents hydrogen, halogen or $C_1$–$C_4$-alkyl and
X represents oxygen or sulphur, characterized in that

a) substituted anilines of the formula (II)

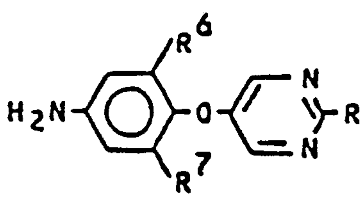 (II)

in which
R, $R^6$ and $R^7$ have the meaning given above, are reacted with benzoyl iso(thio)cyanates of the formula (III)

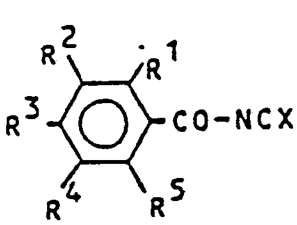 (III)

in which
X, $R^1$, $R^2$, $R^3$, $R^4$ and $R^5$ have the meaning given above, if appropriate in the presence of a diluent, or

b) pyrimidinyloxy-phenyl iso(thio)cyanates of the formula (IV)

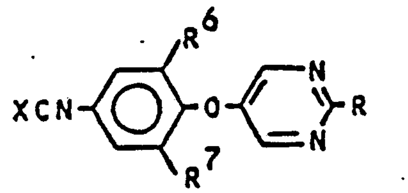 (IV)

in which
X, R, $R^6$ and $R^7$ have the meanings given above, are reacted with benzoic acid amides of the formula (V)

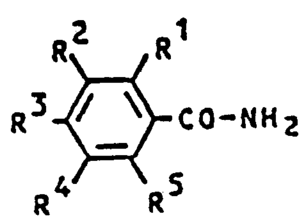 (V)

in which
$R^1$, $R^2$, $R^3$, $R^4$ and $R^5$ have the meanings given above, if appropriate in the presence of a catalyst and, if appropriate, in the presence of a diluent.

5) Pest-combating agents, characterized in that they contain at least one compound of the formula (I) according to Claim 1 or 4.

6) Use of compounds of the formula (I) according to Claim 1 or 4 for combating pests, in particular insects.

7. Process for combating pests, characterized in that compounds of the formula (I) according to Claim 1 or 4 are allowed to act on the pests, preferably insects, or on their habitat.

8) Process for the preparation of pest-combating agents, characterized in that compounds of the formula (I) according to Claim 1 and 4 are mixed with extenders and/or surface-active agents.

9) Compounds of the formula (IX)

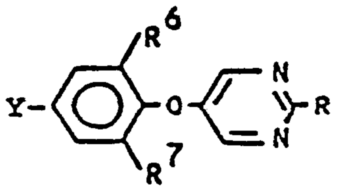

(IX)

in which
R represents hydrogen, $C_1$–$C_6$-alkyl, $C_3$–$C_6$-cycloalkyl, halogeno-$C_1$–$C_6$-alkyl or a phenyl radical which is optionally substituted by halogen, $C_1$–$C_4$-alkyl, $C_1$–$C_4$-alkoxy, $C_1$–$C_4$-alkylthio, halogeno-$C_1$–$C_4$-alkyl, halogeno-$C_1$–$C_4$-alkoxy and/or halogeno-$C_1$–$C_4$-alkylthio,
$R^6$ and $R^7$ are identical or different and represent hydrogen, halogen or $C_1$–$C_4$-alkyl and
Y represents $NO_2$, $NH_2$ or NCX, wherein X denotes oxygen or sulphur.

**Revendications**

1. 3-(pyrimidine-5-yl-oxy-phényl)-1-benzoyl-(thio)urées de formule (I)

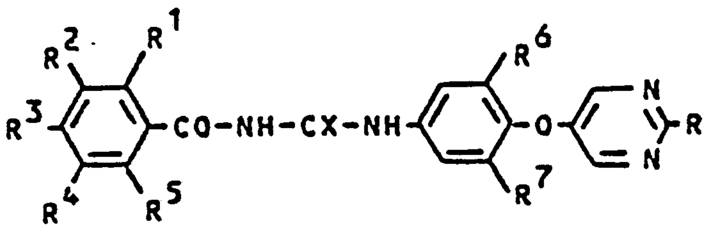

(I)

dans laquelle
R représente l'hydrogène, un groupe alkyle en $C_1$ à $C_6$, cycloalkyle en $C_3$ à $C_6$, halogénalkyle en $C_1$ à $C_6$ ou un reste phényle éventuellement substitué par un halogène, un radical alkyle en $C_1$ à $C_4$, alkoxy en $C_1$ à $C_4$, alkylthio en $C_1$ à $C_4$, halogénalkyle en $C_1$ à $C_4$, halogénalkoxy en $C_1$ à $C_4$ et/ou halogénalkylthio en $C_1$ à $C_4$,
$R^1$ représente l'hydrogène, un groupe nitro, un halogène, un groupe alkyle en $C_1$ à $C_4$ ou alkylthio en $C_1$ à $C_4$,
$R^2$, $R^3$, $R^4$ et $R^5$ sont identiques ou différents et représentent de l'hydrogène ou un halogène;
$R^6$ et $R^7$ sont identiques ou différents et représentent de l'hydrogène, un halogène ou un groupe alkyle en $C_1$ à $C_4$, et
X est l'oxygène ou le soufre.

2. Composés de formule (I) suivant la revendication 1, dans lesquels
R représente l'hydrogène, un groupe alkyle en $C_1$ à $C_4$, cycloalkyle en $C_3$ à $C_6$, halogénalkyle en $C_1$ à $C_4$ ou un reste phényle éventuellement substitué une ou plusieurs fois par un radical alkyle en $C_1$ ou $C_2$, alkoxy en $C_1$ ou $C_2$, alkylthio en $C_1$ ou $C_2$, halogénalkyle en $C_1$ ou $C_2$, halogénalkoxy en $C_1$ ou $C_2$, halogénalkylthio en $C_1$ ou $C_2$, du fluor, du chlore et/ou du brome,
$R^1$ représente l'hydrogène, le fluor, le chlore, le brome, l'iode, un groupe nitro, alkyle en $C_1$ à $C_4$ alkylthio en $C_1$ à $C_4$,
$R^2$, $R^3$, $R^4$ et $R^5$ sont identiques ou différents et représentent l'hydrogène, le fluor, le chlore ou le brome,
$R^6$ et $R^7$ sont identiques ou différents et représentent l'hydrogène, le fluor, le chlore, le brome ou un groupe alkyle en $C_1$ à $C_4$ et
X représente l'oxygène ou le soufre.

3. Composés de formule (I) suivant la revendication 1, dans lesquels
R représente l'hydrogène, un groupe méthyle, éthyle, n-propyle, iso-propyle, n-butyle, sec.-butyle, iso-butyle, tertio-butyle, cyclopropyle, cyclohexyle ou un reste phényle éventuellement substitué une à trois fois par un radical méthyle, éthyle, trifluorométhyle, trifluorométhoxy, trifluorométhyltio, du fluor, du chlore et/ou du brome,
$R^1$ représente le fluor, le chlore, le brome ou l'iode,
$R^2$, $R^3$, $R^4$ et $R^5$ sont identiques ou différents et représentent l'hydrogène, le fluor, le chlore ou le brome,
$R^6$ et $R^7$ sont identiques ou différents et représentent l'hydrogène, le chlore ou un groupe méthyle et
X est l'oxygène ou le soufre.

4. Procédé de préparation de 3-(pyrimidine-5-yl-oxy-phényl)-1-benzoyl-(thio) urées de formule (I)

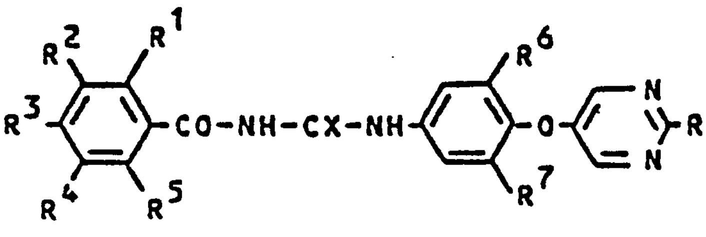

(I)

(I)

dans laquelle
R est l'hydrogène, un groupe alkyle en $C_1$ à $C_6$, cycloalkyle en $C_3$ à $C_6$, halogénalkyle en $C_1$ à $C_6$ ou un reste phényle éventuellement substitué par un halogène, un radical alkyle en $C_1$ à $C_4$, alkoxy en $C_1$ à $C_4$, alkylthio en $C_1$ à $C_4$, halogénalkyle en $C_1$ à $C_4$,

halogénalkoxy en $C_1$ à $C_4$ et/ou halogénalkylthio en $C_1$ à $C_4$,
$R^1$ est l'hydrogène, un groupe nitro, un halogène, un groupe alkyle en $C_1$ à $C_4$ ou alkylthio en $C_1$ à $C_4$,
$R^2$, $R^3$, $R^4$ et $R^5$ sonbt identiques ou différents et représentent l'hydrogène ou un halogène;
$R^6$ et $R^7$ sont identiques ou différents et représentent l'hydrogène, un halogène, ou un groupe alkyle en $C_1$ à $C_4$, et
X est l'oxygène ou le soufre, caractérisé en ce que

a) on fait réagir des anilines substituées de formule (II),

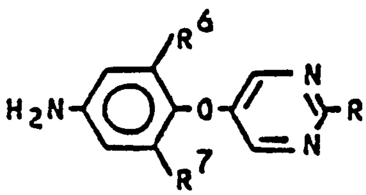

(II)

dans laquelle
R, $R^6$ et $R^7$ ont la définition indiquée ci-dessus, avec des benzoyl-iso(thio)cyanates de formule (III),

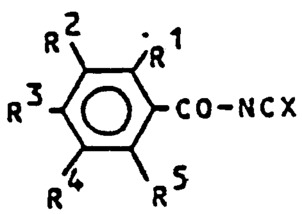

(III)

dans laquelle
X, $R^1$, $R^2$, $R^3$, $R^4$ et $R^5$ ont la définition indiquée ci-dessus,
le cas échéant en présence d'un diluant, ou bien

b) on fait réagir des pyrimidinyloxy-phényl-iso(thio)cyanates de formule (IV),

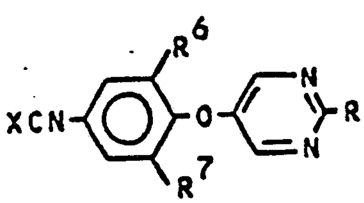

(IV)

dans laquelle
X, R, $R^6$ et $R^7$ ont les définitions indiquées ci-dessus, avec des benzamides de formule (V),

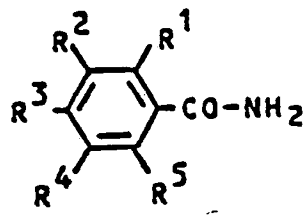

(V)

dans laquelle
$R^1$, $R^2$, $R^3$, $R^4$ et $R^5$ ont les définitions indiquées ci-dessus, le cas échéant en présence d'un catalyseur et en la présence éventuelle d'un diluant.

5. Compositions pesticides, caractérisées par une teneur en au moins un composé de formule (I) suivant la revendication 1 ou 4.

6. Composés de formule (I) suivant la revendication 1 ou 4 pour combattre des parasites, notamment des insectes.

7. Procédé de lutte contre des parasites, caractérisé en ce qu'on fait agir des composés de formule (I) suivant la revendication 1 ou 4 sur des parasites, de préférence des insectes, ou sur leur habitat.

8. Procédé de préparation de compositions pesticides, caractérisé en ce qu'on mélange des composés de formule I suivant la revendication 1 ou 4 avec des diluants et/ou des agents tensioactifs.

9. Procédé de formule (IX)

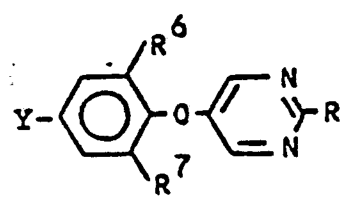

(IX)

dans laquelle
R représente l'hydrogène, un groupe alkyle en $C_1$ à $C_6$, cycloalkyle en $C_3$ à $C_6$, halogénalkyle en $C_1$ à $C_6$, ou un reste phényle éventuellement substitué par un halogène, un radical alkyle en $C_1$ à $C_4$, alkoxy en $C_1$ à $C_4$, alkylthio en $C_1$ à $C_4$, halogénalkyle en $C_1$ à $C_4$, halogénalkoxy en $C_1$ à $C_4$ et/ou halogénalkylthio en $C_1$ à $C_4$,
$R^6$ et $R^7$ sont identiques ou différents et représentent l'hydrogène, un halogène ou un groupe alkyle en $C_1$ à $C_4$ et représente $NO_2$, $NH_2$ ou NCX, où X est l'oxygène ou le soufre.